(19) Europäisches Patentamt
European Patent Office
Office européen des brevets

(11) Publication number: **0 031 909**
**B1**

# (12) EUROPEAN PATENT SPECIFICATION

(45) Date of publication of patent specification: **25.05.83**

(21) Application number: **80107671.2**

(22) Date of filing: **05.12.80**

(51) Int. Cl.³: **C 07 C 49/707,**
C 07 C 49/743,
C 07 C 49/747,
C 07 C 45/59

## (54) 3-Hydroxy-4-cyclopentenones and process for their production.

(30) Priority: **06.12.79 JP 158817/79**
**16.05.80 JP 65771/80**
**11.06.80 JP 79214/80**
**26.09.80 JP 134825/80**

(43) Date of publication of application:
**15.07.81 Bulletin 81/28**

(45) Publication of the grant of the patent:
**25.05.83 Bulletin 83/21**

(84) Designated Contracting States:
**BE CH DE FR GB IT LI NL SE**

(56) References cited:
EP - A - 0 010 761
GB - A - 1 099 382
GB - A - 1 538 736

**CHEMICAL ABSTRACTS vol. 89, 1978 Columbus, Ohio, USA TATSUYA SHONO et al "Cyclopentenone derivatives", page 554, abstract no. 129107g**

(73) Proprietor: **SUMITOMO CHEMICAL COMPANY, LIMITED**
**15 Kitahama 5-chome Higashi-ku**
**Osaka-shi Osaka-fu (JP)**

(72) Inventor: **Saito, Kenji**
**2-10-3-321, Sonehigashi-machi**
**Toyonaka Osaka (JP)**
Inventor: **Yamachika, Hiroshi**
**2-1, Kuwata-cho**
**Ibaraki Osaka (JP)**
Inventor: **Minai, Masayoshi**
**1606-5, Harimada-cho**
**Moriyama Shiga (JP)**

(74) Representative: **Vossius Vossius Tauchner Heunemann Rauh**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 031 909 B1

Courier Press, Leamington Spa, England

3-hydroxy-4-cyclopentenones and process for their production

The present invention relates to a process for producing 3-oxocyclopentenes. More particularly, it relates to a novel and improved process for preparing 3-oxocyclopentenes of the formula:

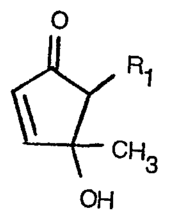

(I)

wherein

$R_1$ is an alkyl group having not more than 6 carbon atoms (e.g. methyl, ethyl, propyl, pentyl, hexyl, cyclohexyl), an alkenyl group having not more than 6 carbon atoms (e.g. allyl, 2-cis-pentenyl, 2-trans-pentenyl, 4-pentenyl, 2,4-cis-pentadienyl), an alkynyl group having not more than 6 carbon atoms (e.g. propargyl, 2-pentynyl) or a group of the formula:

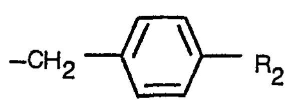

wherein

$R_2$ is a hydrogen atom, a methyl group or a halogen atom (e.g. chlorine, bromine, fluorine).

The 3-oxocyclopentenes of the formula (I) are useful as intermediates for production of agricultural chemicals, medicines (e.g. prostaglandin) and perfumes (e.g. jasmone).

For preparation of the 3-oxocyclopentenes (I), there has been hitherto adopted a method comprising the following reaction [G. Piancatelli et al.: Tetrahedron, *34,* 2775—2778 (1978)]:

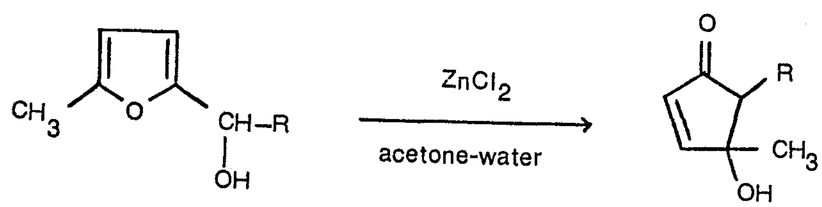


(A)

(B)

However, the applicability of this method is much influenced by the kind of the substituent R. When, for instance, it represents phenyl, 2-thienyl or p-tolyl, the 3-oxocyclopentene (B) is readily obtainable in a good yield within a short reaction time. When, for instance, it represents lower alkyl, lower alkenyl, lower alkynyl, benzyl or substituted benzyl, a long reaction time is required, and yet the yield of the objective 3-oxocyclopentene (B) is extremely low.

As the result of an extensive study, it has now been unexpectedly found that the treatment of the furancarbinol (A) in an aqueous medium within a certain specific pH range can afford the corresponding 3-oxocyclopentene (B) in a good yield within a short reaction time even when the furan-carbinol (A) is of the kind to which the said known medium could be hardly applied.

According to the present invention, there is provided a process for preparing the 3-oxocyclopentenes (I) from the corresponding furan-carbinols of the formula:

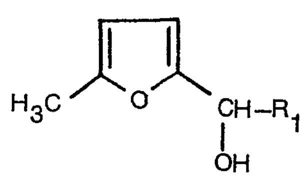

(II)

wherein

$R_1$ is as defined above, characterized in that the latter is treated in an aqueous medium at a pH of 3 to 6.5.

The characteristic feature of the process of this invention resides in that the treatment is effected in an aqueous medium at a pH of 3 to 6.5. As the aqueous medium, there may be used water alone or water containing a small proportion of any organic solvent (e.g. benzene, toluene, xylene, diisopropyl ether, acetone, tetrahydrofuran, dioxane, ethylene glycol). The amount of the aqueous medium to be used may be usually from 0.5 to 200 parts by weight, preferably from 5 to 100 parts by weight, to one part by weight of the starting furan-carbinol (II).

The pH value of the reaction system is required to be maintained between 3 and 6.5, preferably between 3 and 6, the most preferably between 3.5 and 5.8. In case of the pH value being higher than the said upper limit, the reaction rate becomes markedly small or slow. In case of the pH value being lower than the said lower limit, side reactions proceed so that the yield of by-products is increased. As the acidic and/or basic substance for regulation of the pH value, any usual acidic or basic substance may be employed. Examples of the acidic substance are inorganic acids (e.g. sulfuric acid, hydrochloric acid, nitric acid), organic acids (e.g. acetic acid, p-toluenesulfonic acid), acidic metal salts (e.g. sodium dihydrogenophosphate, sodium hydrogensulfite), acid ion-exchange resins. Examples of the basic substance are hydroxides of alkali metals (e.g. sodium, potassium) and alkaline earth metals (e.g. calcium, barium), basic salts of said metals such as carbonates, bicarbonates and acetates, amines (e.g. triethylamine, pyridine), basic ion-exchange resins. Buffer solutions containing these acidic or basic substances are also usable.

The treatment in the process of this invention is usually carried out at a temperature of 20 to 120°C, preferably of 80 to 100°C.

On the treatment, a metal salt, a surfactant or a quaternary ammonium salt may be incorporated into the reaction system for the purpose of shortening the reaction time, enhancing the yield, improving the volume efficiency. Examples of the metal salt are magnesium salts (e.g. magnesium chloride, magnesium bromide, magnesium sulfate, magnesium nitrate, magnesium acetate), manganese salts (e.g. manganese chloride, manganese nitrate), copper salts (e.g. copper sulfate, copper acetate), zinc salts (e.g. zinc chloride), cobalt salts, iron salts, nickel salts. The amount of the metal salt is usually from 0.001 to 0.2 mol, preferably from 0.01 to 0.05 mol, to 1 mol of the furan-carbinol (II). As the surfactant, any of cationic, nonionic and amphoteric ones may be employed. The amount of the surfactant is usually from 0.1 to 20% by weight, preferably from 1 to 5% by weight on the weight of the furan-carbinol (II). Examples of the quaternary ammonium salt are tetrabutylammonium bromide, benzyltrimethylammonium chloride, tricaprylmethylammonium chloride, dodecyltrimethylammonium chloride, caprylbenzyldimethylammonium chloride. Its amount may be from 0.001 to 5 parts by weight to one part by weight of the furan-carbinol (II).

Practically, the treatment of this invention may be carried out by dissolving or suspending the furan-carbinol (II) into an aqueous medium optionally containing any of a metal salt, a surfactant and a quaternary ammonium salt, elevating the temperature to a preferred one and allowing the reaction to proceed while controlling the pH value between 3 and 6.5. When the production amount of the 3-oxocyclopentene (I) reaches the maximum, the reaction is finished.

The starting furan-carbinol (II) may be prepared, for instance, by the reaction of 5-methylfurfural with a Grignard's reagent of the formula: $R_1MX$ wherein $R_1$ is as defined above, M is Mg, Zn or $Al_{2/3}$ and X is a halogen atom.

The present invention will be hereinafter explained further in detail by the following Examples.

Example 1

In a reaction vessel, water (1 liter) and 5-methyl-2-furylallylcarbinol (25 g) were charged, and the pH value was adjusted to 5.5 with an aqueous 1 N NaOH solution and an aqueous 1 N HCl solution. The temperature was elevated up to 100°C to reflux, and the mixture was stirred under reflux for 12 hours while maintaining a pH value of 5.0 to 5.5 by the addition of an aqueous 1 NaOH solution and an aqueous 1 N HCl solution. After cooling to 40°C, the reaction mixture was neutralized with an aqueous 1 N NaOH solution, and sodium chloride (300 g) was added thereto. The mixture was extracted with toluene (100 ml) five times. From the extract, toluene was removed by distillation at 60°C under reduced pressure to give an oily substance (23 g), which was subjected to distillation under reduced pressure to obtain 2-allyl-3-hydroxy-3-methyl-4-cyclopentenone (21.3 g). Yield, 85.2%. B.P., 77°C/0.1 mmHg. NMR spectrum ($CCl_4$, internal standard TMS, $\delta$ ppm, 60 MHz): 7.32 (d, 1H, 4—H); 5.92 (d, 1H, 5—H); 5.81 (complex m, 1H, $—CH_2C\mathit{H}{=}CH_aH_b$); 5.12 (m, 1H, $—CH_2CH{=}C\mathit{H}_aH_b$); 4.93 (m, 1H, $—CH_2—CH{=}CH_a\mathit{H}_b$); 4.12 (broad s, 1H, 3—$\mathit{OH}$); 2.37 (m, 3H, 2—H, s, $—C\mathit{H}_2—CH{=}CH_aH_b$); 1.28 (s, 3H, 3—$C\mathit{H}_3$).

Example 2

In a reaction vessel, water (1600 ml), anhydrous sodium acetate (0.4 g) and 5-methyl-2-furylpropargylcarbinol (20 g) were charged, and the temperature was elevated to reflux. The pH value was adjusted to 4.5 with an aqueous 0.5 N acetic acid solution, and the mixture was stirred under

reflux for 13 hours while maintaining a pH value of 4.0 to 5.0 by the addition of an aqueous 1/3 N NaOH solution. Then, the reaction mixture was cooled to 40°C, neutralized with an aqueous 1/3 N NaOH solution and, after addition of sodium chloride (300 g), extracted with methyl isobutyl ketone (400 ml) five times. From the extract, methyl isobutyl ketone was removed by distillation at 60°C under reduced pressure to give an oily substance (16 g), which was subjected to distillation under reduced pressure to obtain 2-propargyl-3-hydroxy-3-methyl-4-cyclopentenone (14.8 g). Yield, 74%. B.P., 115—120°C/0.1 mmHg. $n_D^{25}$ 1.5124. $C^{13}$ NMR spectrum ($CDCl_3$, internal standard TMS, $\delta$ ppm, 22.5 MHz): 206.2 (1—C); 167.8 (5—C); 130.7 (4—C); 82.0 (—$CH_2$—*C*≡CH); 78.5 (3—C); 70.6 (—$CH_2$—C≡*C*H); 57.0 (2—C); 23.4 (—*C*$H_3$); 14.7 (—*C*$H_2$—C≡CH).

Example 3

In a reaction vessel, water (40 ml) and 5-methyl-2-furyl-n-butylcarbinol (0.5 g) were charged, and the pH value was regulated to 5 with an aqueous 1 N NaOH solution and an aqueous 1 N HCl solution. The temperature was elevated up to 100°C to reflux, and the mixture was stirred under reflux for 30 hours while maintaining a pH value of 5 to 5.5 by the addition of an aqueous 1 N NaOH solution and an aqueous 1 N HCl solution. After cooling to 40°C, the reaction mixture was neutralized with an aqueous 1 N NaOH solution, and sodium chloride (12 g) was added thereto. The mixture was extracted with toluene (40 ml) five times. From the extract, toluene was removed by distillation at 60°C under reduced pressure to give an oily substance (0.48 g), which was subjected to a thin layer chromatography on silica gel with a mixture of ethyl acetate-n-hexane (1:2 by volume) as the developing solvent. The objective part of the silica gel layer was collected by scraping and eluted with ethyl acetate. The silica gel was eliminated by filtration, and the filtrate was concentrated to obtain 2-n-butyl-3-hydroxy-3-methyl-4-cyclopentenone (3.5 g). Yield, 70%. NMR spectrum ($CCl_4$, internal standard TMS, $\delta$ ppm, 60 MHz): 7.25 (d, 1H, 4—H); 5.85 (d, 1H, 5—H); 3.63 (broad s, 1H, 3—*OH*); 2.20 (m, 1H, 2—H); 1.25 (s, 3H, 3—*C*$H_3$).

Examples 4 to 13

Using the furan-carbinol (II), the corresponding 3-oxocyclopentene (I) was prepared in the same manner as in Example 1 under the conditions shown in the following Table:

| Example No. | Furan-carbinol (II) | | Amount of solvent (ml) | pH | Reaction time (hr) | Yield of product *1) (%) |
|---|---|---|---|---|---|---|
| | $R_1$ | Amount (g) | | | | |
| 4 | Methyl | 5 | 100 | 5.0 — 5.5 | 16 | 66 |
| 5 | Ethyl | 5 | 100 | 4.0 — 5.5 | 16 | 68 |
| 6 | Propyl | 0.5 | 40 | 4.0 — 5.3 | 30 | 76 |
| 7 | n-Hexyl | 0.5 | 40 | 4.0 — 5.6 | 30 | 78 |
| 8 | 2-Isoamyl | 0.5 | 40 | 4.0 — 5.5 | 30 | 70 |
| 9 | Isobutyl | 0.5 | 40 | 4.0 — 5.5 | 30 | 68 |
| 10 | 3-Butenyl | 1 | 40 | 4.0 — 5.5 | 30 | 70 |
| 11 | Benzyl | 0.5 | 40 | 4.0 — 5.4 | 30 | 89 |
| 12 | p-Methylbenzyl | 0.5 | 40 | 4.0 — 5.7 | 30 | 85 |
| 13 | p-Chlorobenzyl | 0.5 | 40 | 4.0 — 5.7 | 30 | 88 |

Note: *1) In Examples 6, 7, 12 and 13, isolation was effected by column chromatography.

Example 14

In a reaction vessel, water (750 ml) was charged, and the temperature was elevated up to 100°C to reflux. Then, 5-methyl-2-furylallylcarbinol (24 g) and a solution of $MgCl_2.6H_2O$ (31.5 g) in water (160 ml) were dropwise added thereto in 2.5 hours and 3.5 hours, respectively. During the addition, the pH value of the reaction mixture was maintained at 5.7 to 5.3 by the addition of an aqueous 10% potassium primary phosphate solution and an aqueous 1 N NaOH solution. After about 1.5 hours from the start of the reaction, the pH value became unchanged and remained within said range. After completion of the addition of the aqueous $MgCl_2$ solution, the reaction mixture was stirred at pH 5.5 under reflux at 100°C for further 4.5 hours. Then, the reaction mixture was cooled to 40°C and neutralized with an aqueous 1 N NaOH solution. After addition of sodium chloride (320 g), the mixture was extracted with toluene (250 ml) four times. The extract was dried over anhydrous magnesium sulfate, and toluene was removed therefrom by distillation at 60°C under reduced pressure to give an oily substance (21.6 g), which was subjected to distillation under reduced pressure to obtain 2-allyl-3-hydroxy-3-methyl-4-cyclopentenone (20.4 g). Yield, 85%. B.P., 88—90°C/0.5 mmHg.

Example 15

In a reaction vessel, water (150 ml) and tri-n-octylmethylammonium chloride (450 mg) were charged, and the pH value was adjusted to 5.5 with an aqueous 1/3 N HCl solution and an aqueous 1/3 NaOH solution. Then, 5-methyl-2-furylallylcarbinol (11.0 g) was added thereto, and the temperature was elevated up to 100°C to reflux. The mixture was stirred under reflux for 7 hours while maintaining a pH value of 5 to 5.5 by the addition of an aqueous 1/3 N NaOH solution and an aqueous 1/3 N HCl solution. After cooling to 40°C, sodium chloride (30 g) was added, and the mixture was extracted with toluene (70 ml) five times. From the extract, toluene was removed by distillation at 60°C under reduced pressure to give an oily substance (10.1 g), which was subjected to chromatography on silica gel (100 g) with a mixture of ethyl acetate-n-hexane (1:2 by volume) as the developing solvent to obtain 2-allyl-3-hydroxy-3-methyl-4-cyclopentenone (7.1 g). Yield, 64.5%.

Example 16

In a four-necked flask equipped with a stirrer and a thermometer, 5-methyl-2-furyl-n-pentylcarbinol (1 mol) and water (50 times of the weight of the furan-carbinol) were charged, and the mixture was stirred at 100°C for 20 hours in nitrogen stream while maintaining a pH value of 5.1 to 5.4. After confirming the absence of the starting material by gas chromatography, the reaction mixture was cooled and extracted with toluene. From the organic layer, toluene was removed by distillation to obtain 2-n-pentyl-3-hydroxy-3-methyl-4-cyclopentenone in a yield of 75%. The crude product was purified by column chromatography to obtain pure 2-n-pentyl-3-hydroxy-3-methyl-4-cyclopentenone. $n_D^{21}$ 1.4811.

Example 17

In the same reaction vessel as in Example 16, 5-methyl-2-furyl-(2′-pentynyl)carbinol (0.1 mol), water (50 times of the weight of the furan-carbinol) and an aqueous buffer solution adjusted to pH 5.1 with potassium secondary phosphate and phosphoric acid were added, and the mixture was stirred under heating at 100°C until the starting material was consumed. Then, the reaction mixture was treated as in Example 16 to obtain 2-(2′-pentynyl)-3-hydroxy-3-methyl-4-cyclopentenone in a yield of 84%. The crude product was purified by distillation to obtain pure (2-(2′-pentynyl)-3-hydroxy-3-methyl-4-cyclopentenone. B.P., 100—110°C/0.3—0.5 mmHg. This substance became crystalline after allowed to stand. M.P., 63—65°C.

Example 18

In the same reaction vessel as in Example 16, 5-methyl-2-furyl-(2′-cis-pentenyl)carbinol (1 mol) and water (40 times of the weight of the furan-carbinol) were charged, and the mixture was stirred under heating at 100°C while maintaining a pH value of 4.9 to 5.3 until the starting material was consumed. Then, the reaction mixture was treated as in Example 16 to give an oily substance, which was purified by distillation to obtain 2-(2′-cis-pentenyl)-3-hydroxy-3-methyl-4-cyclopentenone in a yield of 74%. B.P., 97—105°C/0.2—0.4 mmHg. $n_D^{21}$ 1.4820.

Example 19

In the same reaction vessel as in Example 16, 5-methyl-2-furyl-(2′-trans-pentenyl)carbinol (1 mol), water (40 times of the weight of the furan-carbinol) and tetrabutylammonium bromide (1/50 times of the weight of the furan-carbinol) were charged, and the mixture was stirred under heating at 100°C while maintaining a pH value of 5.1 to 5.4 until the starting material was consumed. Then, the reaction mixture was treated as in Example 16 to give an oily substance, which was purified by distillation to obtain 2-(2′-trans-pentenyl)-3-hydroxy-3-methyl-4-cyclopentenone in a yield of 76%. B.P., 105—115°C/0.2 mmHg.

Example 20

In an autoclave, 5-methyl-2-furyl-(2′,4′-cis-pentadienyl)carbinol (0.5 mol), water (40 times of the weight of the furan-carbinol) and an aqueous buffer solution adjusted to pH 5.0 with potassium secondary phosphate and phosphoric acid were charged, and the mixture was stirred under heating at 110°C for 20 hours. The reaction mixture was treated as in Example 16 to obtain 2-(2′,4′-cis-pentadienyl)-3-hydroxy-3-methyl-4-cyclopentenone in a yield of 70%.

Examples 21 to 24

The reaction was carried out in the same manner as in Example 16 but changing the reaction conditions as shown in the following Table. The results are also shown therein.

| Example No. | Catalyst | | Solvent | | pH | Yield (%) |
|---|---|---|---|---|---|---|
| | Kind | Amount *1) | Kind | Amount *1) | | |
| 21 | Magnesium chloride | 1/5 parts by weight | Water | 35 parts by weight | 5.1 – 5.4 | 79 |
| 22 | "Emulgen 910" (polyoxyethylene nonyl phenyl ether) | 1/10 parts by weight | Water | 40 parts by weight | 5.1 – 5.4 | 77 |
| 23 | Tetra-n-butyl-ammonium bromide | 1/50 parts by weight | Water | 40 parts by weight | 5.1 – 5.4 | 78 |
| 24 | – | – | Water<br>Ethylene glycol | 40 parts by weight<br>1/3 parts by weight | 5.1 – 5.4 | 82 |

Note: *1) based on 1 part by weight of the furan-carbinol.

Examples 25 and 26

The reaction was carried out in the same manner as in Example 16 but changing the reaction conditions as shown in the following Table. The results are also shown therein.

| Example No. | Catalyst | | Solvent | | pH | Yield (%) |
|---|---|---|---|---|---|---|
| | Kind | Amount *1) | Kind | Amount *1) | | |
| 25 | "Emulgen 910" | 1/30 parts by weight | Water<br>Ethylene glycol | 30 parts by weight<br>5 parts by weight | 5.0 – 5.3 | 70 |
| 26 | Magnesium chloride | 1/20 parts by weight | Water | 40 parts by weight | 4.9 – 5.2 | 78 |

Note: *1) based on 1 part by weight of the furan-carbinol.

**Claims**

1. A process for producing 3-oxocyclopentenes of the general formula I

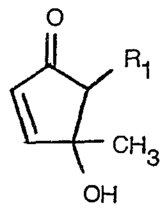

(I)

wherein

$R_1$ is an alkyl group having not more than 6 carbon atoms, an alkenyl group having not more than 6 carbon atoms, an alkynyl group having not more than 6 carbon atoms or a group of the formula:

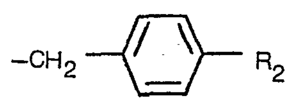

in which $R_2$ is a hydrogen atom, a methyl group or a halogen atom, characterized by treating a furan-carbinol of the general formula II

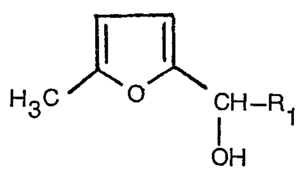

(II)

wherein

$R_1$ is as defined above in an aqueous medium in the presence or absence of a catalyst at a pH of 3 to 6.5 to give the corresponding 3-oxocyclopentene.

2. The process according to claim 1, wherein the treatment is effected at a temperature of 20 to 120°C.

3. The process according to claim 2, wherein the catalyst is a metal salt, a surfactant or a quaternary ammonium salt.

4. A 3-oxocyclopentene of the formula:

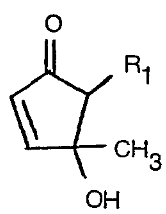

wherein

$R_1$ is pentyl, propargyl, 2-pentynyl, 2-cis-pentenyl, 2-trans-pentenyl or 2,4-cis-pentadienyl.

**Revendications**

1. Procédé de production d'oxo-3 cyclopentène de la formule générale I

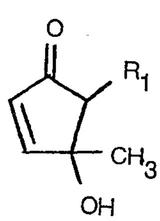

(I)

dans laquelle $R_1$ représente un groupe alcoyle ne possédant pas plus de 6 atomes de carbone, un groupe alcényle ne possédant pas plus de 6 atomes de carbone, un groupe alcynyle ne possédant pas plus de 6 atomes de carbone ou un groupe de la formule

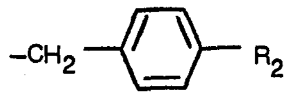

dans laquelle $R_2$ représente un atome d'hydrogène, le radical méthyle ou un atome d'halogène, caractérisé en ce que l'on traite un furanne-carbinol de la formule générale II

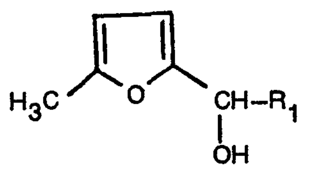

(II)

dans laquelle $R_1$ possède les significations qui lui ont été attribuées ci-dessus, dans un milieu aqueux, en présence ou en l'absence d'un catalyseur, à un pH de 3 à 6,5, de façon à obtenir l'oxo-3 cyclopentène correspondant.

2. Procédé suivant la revendication 1, caractérisé en ce que le traitement s'effectue à une température de 20 à 120°C.

3. Procédé suivant la revendication 2, caractérisé en ce que le catalyseur est un sel de métal, un surfactif ou un sel d'ammonium quaternaire.

4. Oxo-3 cyclopentène de la formule:

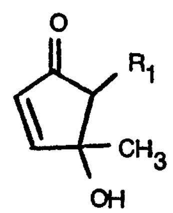

dans laquelle $R_1$ représente un radical pentyle, propargyle, pentyne-2 yle, cis-pentène-2 yle, transpentène-2 yle ou cis-pentadiène-2,4 yle.

**Patentansprüche**

1. Ein Verfahren zur Herstellung von 3-Oxocyclopentenen der allgemeinen Formel I

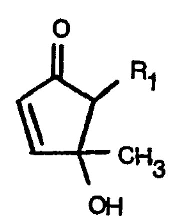

(I)

in der $R_1$ eine Alkylgruppe mit nicht mehr als 6 Kohlenstoffatomen, eine Alkenylgruppe mit nicht mehr als 6 Kohlenstoffatomen, eine Alkinylgruppe mit nicht mehr als 6 Kohlenstoffatomen oder eine Gruppe der Formel

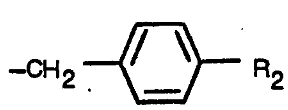

ist, in der $R_2$ ein Wasserstoffatom, eine Methylgruppe oder ein Halogenatom ist, gekennzeichnet durch Behandlung eines Furancarbinols der allgemeinen Formel II

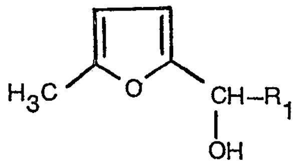

(II)

in der $R_1$ die vorstehend genannte Bedeutung hat, in einem wäßrigen Medium in Gegenwart oder Abwesenheit eines Katalysators bei einem pH-Wert von 3 bis 6,5 unter Bildung des entsprechenden 3-Oxocyclopentens.

2. Das Verfahren nach Anspruch 1, wobei die Behandlung bei einer Temperatur von 20 bis 120°C durchgeführt wird.

3. Das Verfahren nach Anspruch 2, wobei der Katalysator ein Metallsalz, eine grenzflächenaktive Substanz oder ein quarternäres Ammoniumsalz ist.

4. Ein 3-Oxocyclopenten der Formel

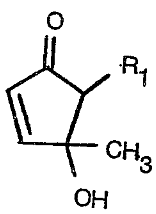

in der $R_1$ Pentyl, Propargyl, 2-Pentinyl, 2-cis-Pentenyl, 2-trans-Pentenyl oder 2,4-cis-Pentadienyl ist.